# EUROPEAN PATENT SPECIFICATION

(11) **EP 2 571 347 B1**
(45) Date of publication and mention of the grant of the patent: **16.10.2024**
(21) Application number: 11722368.5
(22) Date of filing: 18.05.2011
(51) Int. Cl.: A01H 5/10, A01H 6/34, A01H 5/08

(54) **RESISTANCE TO POST HARVEST DETERIORATION IN CUCUMBER**
RESISTENZ GEGEN DEN NACHERNTEVERDERB VON GURKEN
RÉSISTANCE À LA DÉGRADATION POST-RÉCOLTE DU CONCOMBRE

(30) Priority: 19.05.2010 NL 2004748
(43) Date of publication of application: 27.03.2013
(73) Proprietor: Rijk Zwaan Zaadteelt en Zaadhandel B.V., 2678 KX De Lier (NL)
(72) Inventor: DIRKS, Robert, Helene, Ghislain, NL-4731 GL Oudenbosch (NL); VAN DUN, Cornelis, Maria, Petrus, NL-4708 CC Roosendaal (NL); VELTEROP, Joyce, Sylvia, NL-2286 HM Rijswijk (NL); KLOET, Johannes, Willem, NL-2751 CT Moerkapelle (NL)
(74) Representative: van Someren, Petronella F. H. M.
(86) International application number: PCT/EP2011/058077
(87) International publication number: WO 2011/144672

(56) References cited:
- WO-A1-03/018627
- WO-A1-2007/042070
- WO-A1-2007/077230
- WO-A1-2010/057960
- US-A1- 2006 200 875
- S. MARCO ET AL: "Involvement of ethylene in the suppression of hypocotyl elongation in CMV-infected cucumbers", PHYSIOLOGICAL PLANT PATHOLOGY, vol. 8, no. 1, 1 January 1976 (1976-01-01), US, pages 1 - 7, XP055451738, ISSN: 0048-4059, DOI: 10.1016/0048-4059(76)90002-3
- WEHNER T C ET AL: "SCREENING THE CUCUMBER GERMPLASM COLLECTION FOR FRUIT STORAGE ABILITY", HORTSCIENCE, AMERICAN SOCIETY OF HORTICULTURAL SCIENCE, ALEXANDRIA, VA, US, vol. 35, no. 4, 1 July 2000 (2000-07-01), pages 699 - 707, XP009066475, ISSN: 0018-5345
- YAMASAKI SEIJI ET AL: "The M locus and ethylene-controlled sex determination in andromonoecious cucumber plants", PLANT AND CELL PHYSIOLOGY, JAPANESE SOCIETY OF PLANT PHYSIOLOGISTS, JP, vol. 42, no. 6, 1 June 2001 (2001-06-01), pages 608 - 619, XP002566037, ISSN: 0032-0781, DOI: 10.1093/PCP/PCE076
- SEJI YAMASAKI ET AL: "The ethylene-regulated expression of CS-ETR2 and CS-ERS genes in cucumber plants and their possible involvement with sex expression in flowers", PLANT AND CELL PHYSIOLOGY, JAPANESE SOCIETY OF PLANT PHYSIOLOGISTS, JP, vol. 41, no. 5, 1 May 2000 (2000-05-01), pages 608 - 616, XP002566038, ISSN: 0032-0781, DOI: 10.1093/PCP/41.5.608
- HURR B M ET AL: "Developmentally dependent responses of detached cucumber (Cucumis sativus L.) fruit to exogenous ethylene.", POSTHARVEST BIOLOGY AND TECHNOLOGY, vol. 52, no. 2, 2009, DEPARTMENT OF HORTICULTURAL SCIENCES, UNIVERSITY OF FLORIDA, USA, pages 207 - 215, XP002615243, DOI: 10.1016/J.POSTHARVBIO.2008.12.006
- HOVI-PEKKANEN ET AL: "Effects of interlighting on yield and external fruit quality in year-round cultivated cucumber", SCIENTIA HORTICULTURAE, ELSEVIER SCIENCE PUBLISHERS, XX, vol. 116, no. 2, 26 December 2007 (2007-12-26), pages 152 - 161, XP022524546, ISSN: 0304-4238, DOI: 10.1016/J.SCIENTA.2007.11.010

## Description

The present invention relates to cucumber (*Cucumis sativus* L.) plants showing a reduced susceptibility to post harvest deterioration of the cucumber fruits. The invention further relates to parts of the plants, in particular the fruits, to seeds and to other propagation material, and to progeny of the plants.

Breeding of fruity vegetables like cucumber aims at the production of commercial varieties optimally adapted to a professional production environment in order to produce marketable products. Many characteristics need to be taken into account during selection which relate to both input and output traits. One of the most important traits in this respect relates to post harvest quality, in particular to the shelf life of fruits. The avoidance of post harvest deterioration of fruits is an important element that can significantly contribute to an economically more efficient arrangement of the whole production chain.

From the moment of harvest of the cucumber crop until the moment of consumption, the produce is exposed to different exogenous factors contributing to product deterioration. Such factors can be wounding during harvesting and processing, darkness and nutrient deficiency during storage and ethylene during processing, transport and storage. These factors strongly stimulate the post harvest disorders which can become apparent as yellowing or loss of firmness of the fruits. As a consequence of these effects the product becomes much less attractive and thereby unmarketable.

In order to counter the deterioration effects, many post harvest measurements can be taken which reduce these effects. For example, one can store the harvested cucumber fruits at relatively low temperatures of around 10°C to retard senescence. In addition, logistic measurements may be implemented which reduce the transportation time required from the greenhouse or field to the consumer or which prevents the cucumber fruits to be stored in the vicinity of an ethylene source. Furthermore chemical treatments such as with 1-methylcyclopropene (1-MCP) or others may be applied to prevent the post harvest deterioration although food safety and consumer acceptance obviously become an issue.

Many of the post harvest measurements are successful to some extent but there is certainly room for improvement. Moreover, costs involved may be substantial, which is another reason to explore alternatives which reduce the need to apply post harvest treatments. Although knowledge of the physiology of post harvest deterioration is limited, senescence seems to play an important role in this respect.

Senescence is a naturally occurring, developmental process at the end of a life cycle of a plant or plant organ during which metabolism is reprogrammed in order to remobilize resources into reproductive structures like seeds. Yellowing of green leaves or immature green fruits is the most visible symptom of senescence. These are a consequence of chlorophyll breakdown during a relatively late stage of senescence.

Ethylene is an important plant hormone generally known to stimulate physiological processes related to senescence once a leaf or fruit is receptive. In cucumber this stimulation becomes apparent through the yellowing and reduced firmness of harvested fruits. Loss of fruit firmness relates to the enzymatic breakdown of cell wall constituents like pectine, cellulose and hemicellulose. As a consequence the tissue integrity of the fruits is gradually lost which leads to a strong increase of pathogen susceptibility which usually manifests by fungal growth at the fruit surface.

Although fruits of cucumber are known to produce only low amounts of ethylene, especially during the immature phase, the fruits are highly sensitive towards this plant hormone. Therefore, physiological disorders associated with ethylene sensitivity which reduce the post harvest quality of cucumber can be caused by endogenous ethylene synthesis but external sources of ethylene can also be very important in this respect. Exposure to such external sources can occur during harvesting, processing and storage of the produce.

For example, when cucumber fruits are transported or stored in the vicinity of ethylene producing fruits such as apples, pears, bananas or peaches severe deterioration may occur. Furthermore, when cucumber is processed and used as packaged fresh-cut product or in fresh-cut mixtures there may be limitations with respect to the ingredients which can be used due to ethylene release by one or more of the ingredients.

When performing a shelf life test, shelf life can be observed under normal conditions, in air, without any controlled parameters or under controlled parameters such as temperature and light/darkness. However, since deterioration during storage is accelerated in the presence of ethylene, a more severe shelf life test can alternatively be performed with exposure to ethylene.

Although ethylene is the most important plant hormone known to stimulate senescence, other hormones like jasmonate may also contribute to this process.

In addition to playing a role in senescence, ethylene is also known to be involved in many other physiological processes. In cucumber, ethylene plays an important role in the determination of sex expression of the flowers. In general, ethylene treatment of cucumber flower buds enhances femaleness. On the other hand when the sensitivity of female flower buds towards ethylene is reduced for example through the application of silver ions which bind the ethylene binding site of the ethylene receptor protein, the flowers will change their developmental program to enhance the development of male organs. A change in sex expression of the flowers is highly undesirable and must be avoided. Therefore, lowering the sensitivity to ethylene is not perceived as a suitable way of delaying post-harvest deterioration to increase shelf life in cucumbers.

It is the object of the present invention to improve cucumber with respect to post harvest quality, which reduces or eliminates the need to take expensive, preventive measurements which are currently used to maintain the post harvest quality at a high level.

In the research leading to the present invention new cucumber plants were developed the fruits of which have an improved shelf life. It was found that these new plants show a reduced sensitivity to ethylene but surprisingly do not show a change in sex expression.

The present invention thus provides a mutant cucumber *(Cucumis sativus)* plant, which has a monogenic and intermediate genetic trait that is responsible for improved shelf life of the fruits of the plant which is directly linked to reduced sensitivity of the plant to ethylene, wherein the improved shelf life is characterized by:
- harvested cucumber fruits after storage in the presence of ethylene in a concentration of 5 ppm at 21°C in darkness remaining firm after 12 days, wherein firmness is defined by requiring an average force of 5.1 kg/cm² when measuring the fruit flesh at different positions with a penetrometer; and
- harvested cucumber fruits after storage in the presence of ethylene in a concentration of 5 ppm at 21°C in darkness remaining visibly free of fungal growth after 12 days;
- harvested cucumber fruits after storage in the presence of ethylene in a concentration of 5 ppm at 21°C in darkness having reduced discoloration as compared to the negative control line after 8 days;

wherein the plant has reduced sensitivity to ethylene when the ratio between the average hypocotyl length of the plant in etiolated seedling stage grown at 21°C in darkness in air containing 10-20 ppm ethylene and the average hypocotyl length of the plant in etiolated seedling
stage grown at 21°C in darkness in air is at least 0.15, preferably 0.19;
wherein the reduced sensitivity to ethylene does not affect sex expression; and
wherein the plant is obtainable by crossing a cucumber plant with a plant grown from cucumber seeds, a representative sample of which was deposited with the NCIMB under NCIMB accession number 41716, and selecting in the F1 progeny of the cross for plants having the improved shelf life and reduced sensitivity to ethylene.

The present invention thus provides a cucumber *(Cucumis sativus)* plant, which has the same genetic trait that causes the reduced sensitivity to ethylene as found in plants grown from seeds of cucumber EX5003 representative seeds of which were deposited under NCIMB accession number 41716.

In one embodiment, the invention relates to a cucumber plant, which has a genetic trait that causes an improved shelf life as compared to a control plant that has a normal shelf life, which plant is obtainable by crossing a cucumber plant with a plant grown from seeds of cucumber EX5003 representative seeds of which were deposited under NCIMB accession number 41716 and selecting in the progeny of the cross for plants showing an improved shelf life.

The improved shelf life is a shelf life that is at least 6 days longer than the shelf life of a standard cucumber variety, in particular a variety or line that is isogenic except for the genetic shelf life trait. The normal shelf life is the shelf life as found in the standard variety.

The trait is monogenic, i.e. caused by a single gene, and inherited in an intermediate way. Therefore, it can already be observed in the first generation of the cross. When intermediate plants of the first cross are selected and crossed again, a part of the resulting F2 is regarded to have an even more improved shelf life, which corresponds to that of plants grown from seeds of cucumber EX5003 representative seeds of which were deposited under NCIMB accession number 41716. It is thus preferred to select for cucumber plants with an improved shelf life in the second generation of the cross (F2).

"Reduced sensitivity or susceptibility" in this context means lower than the sensitivity or susceptibility of a standard cucumber variety, in particular a line or variety that is isogenic except for the genetic trait of the invention. Reduced sensitivity can be determined by performing a seedling test, as explained below. The normal sensitivity or susceptibility is the sensitivity or susceptibility as found in a standard variety, in particular an isogenic variety or line. Reduced sensitivity is exhibited by an ethylene/air ratio of the hypocotyl that is at least 20% higher than the ethylene/air ratio of the hypocotyl of a line or variety that is isogenic except for the genetic trait of the invention.

The trait is monogenic, i.e. caused by a single gene, and inherited in an intermediate way, therefore it can already be observed in the first generation of the cross. When intermediate plants of the first cross are selected and crossed again, a part of the resulting F2 population is regarded to have an even more reduced sensitivity to ethylene, which corresponds to that of plants grown from seeds of cucumber EX5003 representative seeds of which were deposited under NCIMB accession number 41716. It is thus preferred to select for cucumber plants with a reduced sensitivity to ethylene in the second generation of the cross (F2).

The improved shelf life of the fruits of plants of the invention is directly linked to reduced sensitivity of the plant to ethylene. The reduced susceptibility or sensitivity to ethylene according to the invention does not have a detrimental effect on the sex expression of the flowers and is thus a special form of ethylene insensitivity. This reduced sensitivity to ethylene without affecting the sex expression and leading to a better post harvest shelf life is found to be an intermediate trait that can phenotypically already be observed in the first generation of a cross between any cucumber plant and a plant grown from seeds of cucumber EX5003 representative seeds of which were deposited with the NCIMB under NCIMB accession number 41716 that shows the trait as found in the deposited seed, in particular a plant grown from the deposited seed, or any progeny plant thereof that has retained the trait.

The presence of the trait of the invention in a cucumber plant can be phenotypically detected in various ways, in particular in a seedling test as described below or by assessing the shelf life of the cucumber fruits with respect to colour and/or firmness and/or fungal growth on the surface. Also, combinations of these tests can be used.

The reduced sensitivity to ethylene can be determined on the basis of the relative growth of etiolated seedlings grown from the seed under an ethylene containing atmosphere as compared to air (the so-called "seedling test"). If the ratio between the average hypocotyl length of etiolated seedlings grown in darkness in air containing ethylene and the average hypocotyl length of etiolated seedlings grown in darkness in air is higher than 0.12 the seedling and therefore the plant that can be grown therefrom is insensitive to ethylene according to the invention. This ratio is also called the "score in the seedling test". The hypocotyl lengths in air and in ethylene are determined on plants that are genetically the same with respect to the genetic trait of the invention.

In one embodiment, an ethylene insensitive plant of the invention has a score in the seedling test of at least 0.15.

In one embodiment, an ethylene insensitive plant of the invention has a score in the seedling test of at least 0.20.

In one embodiment, an ethylene insensitive plant of the invention has a score in the seedling test of at least 0.25.

In one embodiment, an ethylene insensitive plant of the invention has a score in the seedling test of at least 0.30.

In one embodiment, an ethylene insensitive plant of the invention has a score in the seedling test of at least 0.35.

In one embodiment, an ethylene insensitive plant of the invention has a score in the seedling test of at least 0.40.

The result of the seedling test can also be expressed as percentage difference in hypocotyl length. The values above are then multiplied by 100.

Another phenotypic parameter that is relevant to the post harvest shelf life is the appearance of harvested cucumber fruits after storage in the presence of ethylene. Mutant plants that have the improved shelf life and reduced sensitivity to ethylene of the invention remain firm and free of fungal growth after at least 8 days of exposure to ethylene.

In one embodiment, a plant of the invention with improved shelf life and reduced sensitivity to ethylene remains firm and free of fungal growth after at least 12 days of exposure to ethylene.

A further parameter related to an improved shelf life is the firmness of the fruits as tested with a penetrometer (model FT327, QA Supplies, Norfolk Virginia). The average force expressed as kg/cm² required to penetrate the fruit flesh is significantly higher for fruits of plants of the invention than for control fruits that are sensitive to ethylene. The difference, when tested under ethylene exposure, may vary from an improvement as compared to the control of 0.5 to 0.9 kg/cm² or more after 8-12 days of storage.

Free of fungal growth means that the cucumber fruit shows no visible signs of fungal growth due to post-harvest diseases.

The presence of the genetic information that is responsible for the trait of the invention, i.e. improved shelf life and/or reduced sensitivity to ethylene, in the genome of a plant with improved shelf life and/or reduced sensitivity to ethylene can be determined with the following test. The plant to be tested should be or should be made homozygous for the genetic information responsible for improved shelf life and/or reduced sensitivity to ethylene. Genetic information as used herein is intended to refer to a genetic trait or gene.

This plant is then crossed with a tester plant that carries the genetic information that is responsible for the trait of the invention in homozygous condition. If the plant to be tested has improved shelf life and/or reduced sensitivity to ethylene as a result of the same genetic information that is responsible for the trait of the invention, all progeny plants of the first cross and successive generations will express the trait. If the improved shelf life of the plant to be tested is the result of a different part of the genome, e.g. another gene or locus, segregation will occur. The tester plant can be any plant that carries the genetic information of the invention in homozygous condition, such as plants directly grown from the deposited seeds or progeny thereof that has retained the trait.

When a cucumber plant, the fruits of which show improved shelf life, is crossed with a tester plant grown from seed of *Cucumis sativus* EX5003 as deposited with the NCIMB under accession number NCIMB 41716 which comprises the improved shelf life trait of the invention, or a progeny plant thereof that comprises the improved shelf life trait comprised in *Cucumis sativus* EX5003 as deposited with the NCIMB under accession number NCIMB 41716 or a plant derived from *Cucumis sativus* EX5003 as deposited with the NCIMB under accession number NCIMB 41716 and comprising the improved shelf life trait, plants of the first generation progeny (F1) of said cross show a 1:0 segregation for improved shelf life. In both the tester plant and the plant of the invention the improved shelf life trait is present in homozygous condition. Plants of the second and further generations, if obtained by selfing also show a 1:0 segregation for the improved shelf life. In the context of this application the "improved shelf life trait" is the genetic trait that causes the improved shelf life phenotype.

When a cucumber plant which shows reduced sensitivity to ethylene is crossed with a tester plant grown from seed of *Cucumis sativus* EX5003 as deposited with the NCIMB under accession number NCIMB 41716 which comprises the ethylene insensitivity trait of the invention, or a progeny plant thereof that comprises the ethylene insensitivity trait comprised in *Cucumis sativus* EX5003 as deposited with the NCIMB under accession number NCIMB 41716 or a plant derived from *Cucumis sativus* EX5003 as deposited with the NCIMB under accession number NCIMB 41716 and comprising the ethylene insensitivity trait, plants of the first generation progeny (F1)of said cross show a 1:0 segregation for reduced susceptibility to ethylene. In both the tester plant and the plant of the invention the ethylene insensitivity trait is present in homozygous condition. Plants of the second and further generations, if obtained by selfing also show a 1:0 segregation for the ethylene insensitivity trait. In the context of this application the "ethylene insensitivity trait" is the genetic trait that causes the improved shelf life phenotype.

The improved shelf life of cucumbers of the invention has a genetic basis in the cucumber's genome. With the above described cross with a tester plant, plants can be identified as being plants of the invention.

In a cucumber plant, showing the ethylene insensitivity and/or improved shelf life trait, said trait is suitably introgressed from a plant grown from seed that was deposited with the NCIMB under accession number NCIMB 41716, or progeny thereof.

"Introgression" as used herein is intended to mean introduction of a genetic trait into a plant not carrying the genetic trait by means of crossing and selection in the usual way known to the skilled person.

It is possible to pyramid multiple alleles of reduced susceptibility towards ethylene and/or improved shelf life.

Progeny of the plants as claimed are also part of this invention. "Progeny" as used herein is intended to encompass all plants having the same or a similar improved shelf life phenotype and the corresponding reduced susceptibility towards ethylene phenotype and the same or a similar genetic trait causing this improved shelf life or reduced susceptibility towards ethylene as the original plants described herein and being derived therefrom in any way, such as by crossing, haploid culture, protoplast fusion or other techniques. Such progeny is not only the first but also all further generations as long as the improved shelf life and/or the reduced susceptibility to ethylene is retained, preferably both genetically and phenotypically.

The mutant plants of the invention were obtained by a chemical random mutagenesis procedure combined with an efficient phenotypic selection procedure based on a response of etiolated seedlings to ethylene. Such seedling based selection system is by far more efficient in terms of numbers of plants which can be assessed per man hour as compared to the use of mature cucumber fruits. Furthermore, the time to produce plant material for screening is obviously much more reduced in the case of seedlings as compared to immature fruits. A further advantage of this approach is the use of the selection conditions at the seedling stage as predictive phenotypic marker for the post harvest trait in consecutive generations once a successful event has been identified.

In order to determine the response of the etiolated cucumber seedlings towards ethylene, use was made of especially designed plastic containers in which cucumber seedlings were grown on filter papers under an atmosphere in which ethylene levels can be varied. It was indeed found that the cucumber seedlings responded to the presence of ethylene by a reduced elongation of the hypocotyl which in principle would allow to select for ethylene insensitive mutants in case such mutants reside in the available population and in case the insensitivity is expressed phenotypically at the seedling level under the experimental conditions which were applied.

By growing large numbers of etiolated cucumber seedlings from a population containing randomly induced mutations under an ethylene containing atmosphere, it was thus surprisingly found that seedlings showing reduced ethylene sensitivity as compared to the ethylene sensitivity of the starting population can be obtained. Out of a population of approximately 40.000 M2 seedlings, those seedlings were selected which showed hypocotyl elongation which was significantly larger as compared to the average hypocotyl elongation of the whole population. The 46 seedlings identified in this manner were qualified as being putatively less sensitive to ethylene as compared to the control.

The M2 plants thus selected on the basis of a reduced response to ethylene were used to grow M3 seeds. Subsequently, the inbred lines descending from the ethylene insensitive events which successfully produced M3 seeds were re-evaluated for their response to ethylene. The level of insensitivity of each inbred line was scored on the basis of the relative growth of the seedling under an ethylene containing atmosphere versus air. Based on this criterion 27/31 events were now classified as being confirmed insensitive. Apparently and unexpectedly, genetic variation existed which leads to differences in the average etiolation response depending on the level of ethylene insensitivity. As expected, this insensitivity to ethylene led in most cases to a change in sex-expression of the floral organs, particularly a tendency towards higher maleness. This especially occurred in those events which showed the largest insensitivity response as judged by the length of the etiolated hypocotyls. It was however surprisingly found that, within this collection of ethylene insensitive mutants, also a number of events could be identified which unexpectedly were not affected in sex expression of their floral organs. One of these events, identified as event 15, was used to develop plants of the invention.

The inbred lines which showed a confirmed ethylene insensitivity in the ethylene test and which were not changed in their sex expression were resown and grown in a greenhouse under regular cucumber production conditions to produce immature (but harvest mature) fruits that were harvested and assessed for yellowing under an atmosphere containing ethylene. As negative controls ethylene sensitive plants were grown which originate from the population and which were used to select the ethylene insensitive events.

Surprisingly, the seeds of all ethylene insensitive mutants germinated normally i.e. comparable to the seeds of a near-isogenic ethylene sensitive control plant of the original non-mutated population when planted in potting soil. After cultivation, immature, commercially harvestable, fruits were harvested and exposed to ethylene. One week of post-harvest incubation of fruits at 20-21°C under a 5 ppm ethylene containing atmosphere in the dark resulted in a strong induction of yellowing of the fruits of the ethylene sensitive control plants. However, the ethylene insensitive events which were assessed for fruit quality and which were not affected in sex expression showed a significant reduction of yellowing. In addition, the fruits of the ethylene insensitive mutants were significantly more firm after exposure to ethylene as compared to fruits of ethylene sensitive control plants.

These results surprisingly demonstrate that ethylene insensitivity which was selected for at the seedling level can reduce physiological disorders at the mature plant level, even at the post harvest stage. It is furthermore surprisingly demonstrated that in certain events a relatively low level of ethylene insensitivity does not lead to a change in sex expression in cucumber, but is sufficient to provide a significant improvement of the post harvest quality of the fruits.

The invention further relates to seed of the cucumber plant of the invention and to parts of the plant that are suitable for sexual reproduction, i.e. propagation material. Such parts are for example selected from the group consisting of microspores, pollen, ovaries, ovules, embryo sacs and egg cells. In addition, the invention relates to parts of the plant that are suitable for vegetative reproduction, in particular cuttings, roots, stems, cells, protoplasts, etc.

A tissue culture comprises regenerable cells. Such tissue culture can be derived from leaves, pollen, embryos, cotyledon, hypocotyls, meristematic cells, roots, root tips, anthers, flowers, seeds and stems.

Cells of the plant that carry the genetic trait that causes the improved shelf life and/or reduced sensitivity to ethylene phenotype of the invention can be in isolated form or be part of a plant, of which the fruits show the improved shelf life and/or reduced sensitivity to ethylene as described herein.

Cucumber plants of the invention have the same or similar improved shelf life and/or ethylene insensitivity trait, in particular both the same phenotype and the same genetic trait, as cucumber plants of the invention, of which representative seed was deposited under NCIMB Accession number NCIMB 41716, which plants are grown from seeds of the plant of the invention or regenerated from parts thereof, or from a tissue culture.

The invention also relates to progeny of the cucumber plant of the invention. Such progeny can be produced by sexual or vegetative reproduction of a plant of the invention or a progeny plant thereof. The regenerated progeny plant has the same or similar improved shelf life and/or ethylene insensitivity trait, in particular both the same phenotype and the same genetic trait, as the plant, of which representative seed was deposited under NCIMB Accession number NCIMB 41716. This means that such progeny has the same characteristics as claimed for the cucumber plant of the invention. In addition to this, the plant may be modified in one or more other characteristics. Such additional modifications are for example effected by mutagenesis or by transformation with a transgene.

Also disclosed herein is hybrid seed and a method of producing hybrid seed comprising crossing a first parent plant with a second parent plant and harvesting the resultant hybrid seed, wherein said first parent plant and/or said second parent plant are a plant with the trait of the invention.

It is clear that the parent that provides the trait of the invention is not necessarily a plant grown directly from the deposited seeds. The parent can also be a progeny plant from the seed or a progeny plant from seeds that are identified to have the trait of the invention by other means.

In this application the term "reduced susceptibility to ethylene", "insensitivity to ethylene", "less sensitive to ethylene" and "lower sensitivity to ethylene" are used interchangeably. The terms mean that the effect of ethylene on the post harvest quality of the cucumber fruits formed by plants of the invention is reduced in that the plants have a longer shelf life as illustrated by a longer period during which the cucumber fruits retain their green colour, and/or remain more firm, and do not show fungal growth at their surface for an extended period as compared to a control that does not have the trait of the invention. In addition, the plants of the invention do not show an affected sex expression in spite of their ethylene insensitivity.

"Improved shelf life trait" as used herein is intended to encompass a phenotypically detectable improvement in the shelf life of the fruits as defined herein with respect to colour and firmness. This trait has a genetic basis in the plant and is present in the genome of the plant but is detected in the fruits. The improvement is present when firmness of the fruits is at least 0.5 kg/cm² better, preferably at least 0.6 kg/cm² better, more preferably at least 0.7 kg/cm² better, even more preferably at least 0.8 kg/cm² better, most preferably at least 0.9 kg/cm² better as compared to a control fruit of a standard variety, in particular a cucumber fruit of an isogenic line or variety without the genetic trait of the invention, in a test wherein fruits are incubated post-harvest for 12 days at a temperature between 20 and 21°C in an atmosphere containing 5 ppm ethylene in the dark.

"Ethylene insensitivity trait" as used herein is intended to encompass a score in the seedling test as defined herein of higher than 0.12 (12%), in particular 0.19 (19%) or higher. Intermediate ethylene insensitivity is also intended to encompass a score in the seedling test higher than 0.12 (12%).

The two traits have in fact the same genetic basis but are named herein after the method by which they are assessed.

The present invention will be further illustrated in the Examples that follow and that are given to illustrate but not limit the invention.

In the Examples reference is made to the following figures:

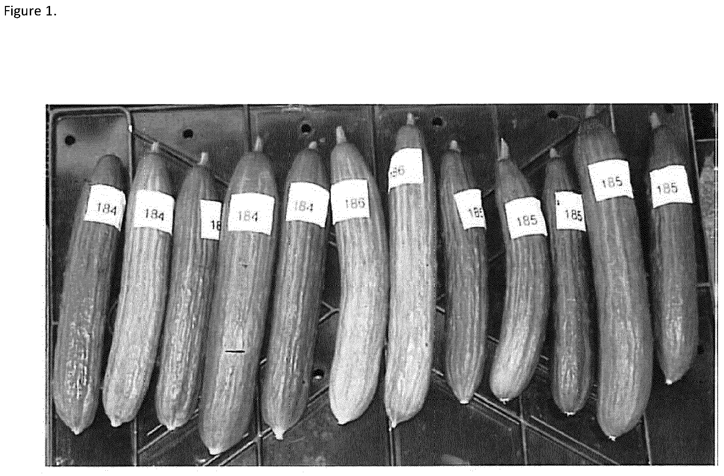

**Figure 1****:** Appearance of harvested cucumber fruits after 8 days exposure to 5 ppm ethylene. The fruits labeled 186 are derived from the negative control line. The fruits labeled 184 and 185 are derived from ethylene insensitive events 15 (EX5003) and 20, respectively. The skins of the fruits of the ethylene insensitive events are darker green as compared to the negative control. Light grey matches yellow, dark grey matches green.

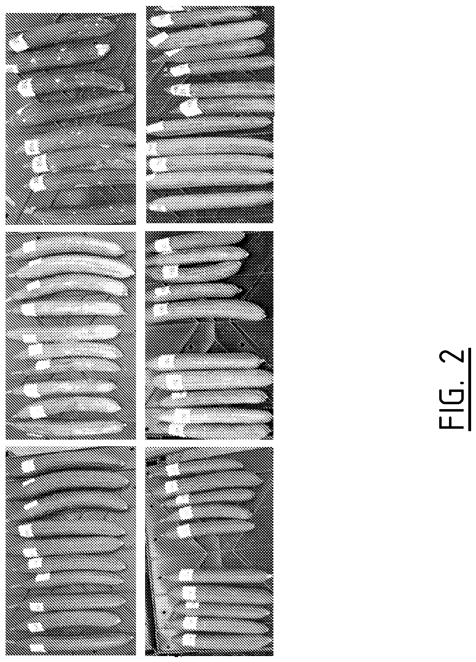

**Figure 2****:** Appearance of harvested cucumber fruits after 0 (left column), 8 (middle column) and 12 (right column) days exposure to 5 ppm ethylene. The negative control fruits (BF11) are depicted in the upper row whereas the fruit from the ethylene insensitive (ethylene R) mutants are shown in the lower row. On the left fruits from event 15 (EX5003) and on the right fruit from event 20 are shown. Light grey matches yellow, dark grey matches green.

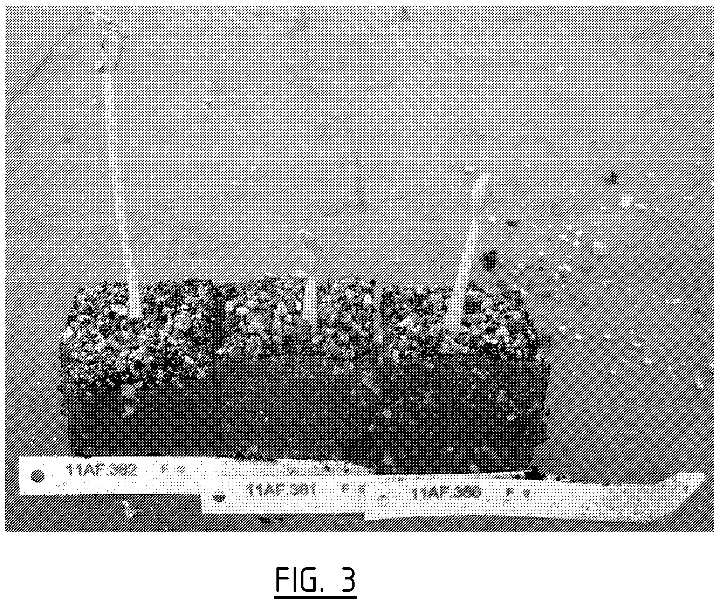

**Figure 3****:** Comparison of hypocotyl lengths of three seedlings. Center: control; right: intermediate ethylene insensitivity (F1(Control x EMS-15)); left: deposited seed (EMS-15).

### Deposit information

Seeds of *Cucumis sativus* EX5003 (which are seeds of the M3 line ID 15 in **Table 1)** were deposited on May 13th 2010 with NCIMB Ltd., Ferguson Building, Craibstone Estate, Bucksburn, Aberdeen, AB21 9YA Scotland, UK and given the accession number NCIMB 41716.

### EXAMPLES

### EXAMPLE 1

### Genetic modification of cucumber by ethyl methane sulfonate (ems)

Seeds (M0) of the cucumber breeding line BF11 were treated with ems by submergence of approximately 5000 seeds into an aerated solution of 0.07% (w/v) ems during 24 hours at room temperature.

The treated M1 seeds were germinated and the resulting M1 plants were grown and self-pollinated in a greenhouse to produce M2 seeds.

After maturation, M2 seeds were harvested and bulked in one pool. The resulting pool of M2 seeds was used as starting material to identify the individual M2 plants that show reduced sensitivity to ethylene.

The efficacy of the genetic modification procedure was assessed by determining the occurrence of bleached plants, which is indicative for chlorophyll loss due to modifications in genes directly or indirectly involved in the formation or accumulation of chlorophyll.

### EXAMPLE 2

### Identification of cucumber plants which have obtained reduced sensitivity to ethylene

M2 cucumber seeds were germinated on paper in a small plastic container with an ethylene concentration of 10-20 vpm (volume parts per million) at 21°C in the dark. 1 Vpm contains 0.41 pmol/litre or 1.14 µg/litre. Ethylene-insensitive mutants were compared to ethylene-sensitive controls, and selected on the basis of an elongated hypocotyl when grown in darkness.

The ethylene-insensitive mutants which were obtained were grown in a greenhouse in order to produce M3-lines by self-fertilisation. In total 31 M3-lines were analysed with the seedling-test to confirm the ethylene insensitivity. When on average the emerged seedlings had produced a longer hypocotyl the event was qualified as being ethylene insensitive. In total 27 out of 31 events were given this qualification. When a line was segregating for ethylene-insensitivity, plants were selected and after an additional cycle of inbreeding a final seedling-test was performed to select homozygous ethylene-insensitive lines.

Ethylene-insensitive mutants were identified by their longer hypocotyls in comparison with sensitive control line, BF11. Results are presented in **Table 1.**

**Table 1: The average hypocotyl length of etiolated seedlings of M3 lines derived from putative ethylene insensitive events (ID column) when grown in darkness in air (air column) or in air containing ethylene (ethylene column). The ratio of the average hypocotyl length between plants grown in ethylene and in air is also shown, as a percentage (% ethylene/air column). The S/R column shows the scores for each of the events as being sensitive to ethylene (S) or insensitive to ethylene (R). The (-) control is the M0 cucumber starting line which is used to produce the M2 population used in the ethylene insensitivity screens described in this application. The hypocotyl lengths in air and ethylene are determined in seeds having the same mutation.**

| **ID** | **air** | **ethylene** | **% ethylene/air** | **S/R** |
|---|---|---|---|---|
| **(-) control** | 10 | 1,0 | 10 | S |
| **2** | 10 | 4,2 | 41 | R |
| **3** | 12 | 3,8 | 32 | R |
| **4** | 12 | 1,0 | 8 | S |
| **5** | 13 | 3,3 | 27 | R |
| **6** | 13 | 4,1 | 32 | R |
| **8** | 10 | 3,2 | 32 | R |
| **9** | 11 | 3,3 | 31 | R |
| **10** | 13 | 1,8 | 15 | R |
| **11** | 12 | 2,0 | 17 | R |
| **12** | 10 | 1,0 | 10 | S |
| **13** | 11 | 2,8 | 26 | R |
| **14** | 10 | 1,7 | 17 | R |
| **15** | 10 | 3,4 | 33 | R |
| **16** | 10 | 2,1 | 20 | R |
| **17** | 10 | 1,5 | 15 | R |
| **18** | 10 | 2,8 | 29 | R |
| **19** | 10 | 2,6 | 27 | R |
| **20** | 17 | 3,3 | 19 | R |
| **21** | 9 | 2,4 | 28 | R |
| **22** | 9 | 2,1 | 25 | R |
| **25** | 10 | 1,9 | 18 | R |
| **30** | 9 | 1,0 | 12 | S |
| **31** | 10 | 2,9 | 29 | R |
| **32** | 10 | 2,8 | 28 | R |
| **33** | 9 | 3,0 | 35 | R |
| **34** | 8 | 1,9 | 23 | R |
| **40** | 10 | 1,0 | 10 | S |
| **42** | 10 | 1,9 | 19 | R |
| **44** | 11 | 2,6 | 24 | R |
| **45** | 9 | 3,8 | 43 | R |
| **46** | 9 | 2,3 | 25 | R |

Based on these results event 15 was selected for the deposit. **Figure 3** shows a comparison between the hypocotyls of a control seedling(center), a cucumber seedling grown from the deposited seeds (left) and a cucumber seedling that shows an intermediate ethylene insensitivity from a cross between the control and the deposited seeds(right).

### EXAMPLE 3

### Identification of ethylene insensitive cucumber plants which have obtained reduced post harvest deterioration with respect to fruit yellowing and fruit firmness

A number of confirmed ethylene insensitive events (at the level of seedling etiolation response in air containing ethylene) which were not changed with respect to sex-expression were evaluated for fruit shelf life when exposed to ethylene. Per event 5-8 fruits were collected and incubated at 21°C in darkness under an atmosphere containing 5 ppm ethylene.

After 8 days, the negative control line BF11 (which represents the genetic background of each of the mutants) turned from green to almost completely yellow as expected. The ethylene insensitive mutants also changed colour but the fruits were less yellow as compared to the control fruits **(****Figure 1****).**

A similar result was obtained when the fruits were compared during a shelf life experiment in air. The shelf life was thus found to be enhanced under different storage conditions.

In addition, fruit firmness under ethylene exposure was determined after 12 days for a number of fruits of each event by cutting the fruit lengthwise in half and subsequently measuring the resistance of the fruit flesh at different positions of the fruit by using a penetrometer. The average force expressed as kg/cm² required to penetrate the fruit flesh was 4.4 for the negative control fruits and ranging from 5.1 to 5.5 for ethylene insensitive fruits. The results which are listed in **Table 2** clearly show that the ethylene insensitive fruits retained a higher level of firmness as compared to the negative control fruits.

**Table 2**

| Shelf life under ethylene exposure | | | | | |
|---|---|---|---|---|---|
| Data are averages from a minimum of 5 fruits, and firmness is tested minimum 4 times per fruit. | | | | | |
| | firmness (12 DAH) kg/cm² | colour* (0 DAH) | colour (8 DAH) | colour (12 DAH) | fungal growth** (12 DAH) |
| Control 1 BF11 | 4.4 | 7 | 3 | 2 | 4 |
| event 15 | 5.1 | 7 | 5 | 4 | 0 |
| event 20 | 5.5 | 7 | 6 | 4 | 0 |

| | | | | | |
|---|---|---|---|---|---|
| DAH = Days After Harvest * colour: lightest/yellow/poor=1, darkest = 10 ** fungal growth: 0 = none, 5 = covered with fungus | | | | | |

Upon manual recording this effect could clearly be ascertained. The negative control fruits were soft and could easily be bended without breaking whereas the ethylene insensitive fruits were still firm. Prolonged exposure up to 12 days to ethylene resulted in further deterioration of the control fruits and as a consequence fungal growth at the surface became visible. The ethylene insensitive fruits, despite losing some of their colour, remained firm and free of fungal growth (**Figure 2**). Therefore, it is concluded that the ethylene insensitivity in cucumber according to the invention is a trait that improves the post harvest quality of cucumbers.

### EXAMPLE 4

### Transfer of the trait of the invention to other cucumbers

The trait of the invention as present in *Cucumis sativus* plants of EX5003, deposited under NCIMB accession number 41716, was crossed into a *Cucumis sativus* plant lacking such trait.

First, plants of EX5003 were crossed with an ethylene sensitive plant: cultivar Roxanna of Rijk Zwaan. Due to the intermediate pattern of inheritance of the trait, the resulting F1 plants already showed an intermediate level of improved shelf life. The F1 plants were selfed in order to produce an F2 with plants comprising the trait in homozygous condition, thus having the same improved shelf life as is present in the plants of EX5003, deposited under NCIMB accession number 41716.

From the selfed F2 population those plants which showed the same level of insensitivity to ethylene were selected by conducting a seedling test as described in example 2.

This way it was possible to transfer the trait of the invention to ethylene sensitive cucumbers thus conferring the improved shelf life to their fruits.

## Claims

1. Mutant cucumber (*Cucumis sativus*) plant, which has a monogenic and intermediate genetic trait that is responsible for improved shelf life of the fruits of the plant which is directly linked to reduced sensitivity of the plant to ethylene,
wherein the improved shelf life is **characterized by**:
- harvested cucumber fruits after storage in the presence of ethylene in a concentration of 5 ppm at 21 °C in darkness remaining firm after 12 days, wherein firmness is defined by requiring an average force of 5.1 kg/cm² when measuring the fruit flesh at different positions with a penetrometer; and
- harvested cucumber fruits after storage in the presence of ethylene in a concentration of 5 ppm at 21°C in darkness remaining visibly free of fungal growth after 12 days;
- harvested cucumber fruits after storage in the presence of ethylene in a concentration of 5 ppm at 21°C in darkness having reduced discoloration as compared to the negative control line after 8 days;
wherein the plant has reduced sensitivity to ethylene when the ratio between the average hypocotyl length of the plant in etiolated seedling stage grown at 21°C in darkness in air containing 10-20 ppm ethylene and the average hypocotyl length of the plant in etiolated seedling stage grown at 21°C in darkness in air is at least 0.15, preferably 0.19;
wherein the reduced sensitivity to ethylene does not affect sex expression; and wherein the plant is obtainable by crossing a cucumber plant with a plant grown from cucumber seeds , a representative sample of which was deposited with the NCIMB under NCIMB accession number 41716, and selecting in the F1 progeny of the cross for plants having the improved shelf life and reduced sensitivity to ethylene.

2. Cucumber plant as claimed in claim 1, wherein the plant is obtainable by selecting in the F2 progeny of the cross that is obtained after crossing the F1 with itself or with another plant for plants having the improved shelf life and reduced sensitivity to ethylene.

3. Seed of a cucumber plant as claimed in claim 1 or 2, wherein the fruit produced by the plant grown from the seed shows the improved shelf life and ethylene insensitivity as defined in claim 1.

4. Progeny of a cucumber plant or cucumber seed as claimed in any one of the claims 1 to 3, wherein the progeny plant has the improved shelf life and ethylene insensitivity as defined in claim 1.

5. Propagation material of a plant as claimed in claim 1, 2 or 4, suitable for sexual reproduction, wherein a plant grown or regenerated from the propagation material has the improved shelf life and ethylene insensitivity as defined in claim 1.

6. Plant part of a plant as claimed in claim 1, 2 or 4, suitable for vegetative reproduction, wherein a plant grown or regenerated from the plant part has the improved shelf life and ethylene insensitivity as defined in claim 1.

## Patentansprüche

1. Mutanten-Gurken-Pflanze *(Cucumis sativus),* die über ein monogenes und intermediäres genetisches Merkmal verfügt, das für eine verbesserte Haltbarkeit der Früchte der Pflanze verantwortlich ist, was wiederum in direktem Zusammenhang mit einer verringerten Empfindlichkeit der Pflanze gegenüber Ethylen steht,
wobei die verbesserte Haltbarkeit **gekennzeichnet ist durch:**
- geerntete Gurkenfrüchte bleiben nach Lagerung in Gegenwart von Ethylen bei einer Konzentration von 5 ppm bei 21 °C im Dunkeln nach 12 Tagen fest, wobei die Festigkeit **durch** eine erforderliche durchschnittliche Kraft von 5,1 kg/cm² bei der Messung des Fruchtfleisches an verschiedenen Stellen mit einem Penetrometer definiert ist, und
- geerntete Gurkenfrüchte blieben nach Lagerung in Gegenwart von Ethylen bei einer Konzentration von 5 ppm bei 21 °C im Dunkeln nach 12 Tagen sichtbar frei von Pilzbefall;
- geerntete Gurkenfrüchte weisen nach Lagerung in Gegenwart von Ethylen bei einer Konzentration von 5 ppm bei 21 °C im Dunkeln nach 8 Tagen eine geringere Verfärbung im Vergleich zur negativen Kontrolllinie auf;
wobei die Pflanze eine verringerte Empfindlichkeit gegenüber Ethylen aufweist, wenn das Verhältnis zwischen der durchschnittlichen Hypokotyllänge der Pflanze im etiolierten Sämlingsstadium, die bei 21 °C im Dunkeln in Luft mit 10-20 ppm Ethylen gewachsen ist, und der durchschnittlichen Hypokotyllänge der Pflanze im etiolierten Sämlingsstadium, die bei 21 °C im Dunkeln in Luft gewachsen ist, mindestens 0,15, vorzugsweise 0,19, beträgt;
wobei die verringerte Empfindlichkeit gegenüber Ethylen keinen Einfluss auf die Geschlechtsausprägung hat; und
wobei die Pflanze **durch** Kreuzung einer Gurkenpflanze mit einer aus Gurkensamen gezogenen Pflanze erhältlich ist, von der eine repräsentative Probe beim NCIMB unter der NCIMB-Zugangsnummer 41716 hinterlegt wurde, und **durch** Selektion von Pflanzen mit verbesserter Haltbarkeit und geringerer Ethylenempfindlichkeit in der F1-Nachkommenschaft der Kreuzung.

2. Gurkenpflanze nach Anspruch 1, wobei die Pflanze erhältlich ist, indem in der F2-Nachkommenschaft der Kreuzung, die nach der Kreuzung der F1 mit sich selbst oder mit einer anderen Pflanze erhalten wird, Pflanzen mit verbesserter Haltbarkeit und verringerter Ethylenempfindlichkeit selektiert werden.

3. Samen einer Gurkenpflanze nach Anspruch 1 oder 2, wobei die von der aus dem Samen gezogenen Pflanze produzierten Früchte die in Anspruch 1 definierte verbesserte Haltbarkeit und Ethylenunempfindlichkeit aufweisen.

4. Nachkommen einer Gurkenpflanze oder eines Gurkensamens nach einem der Ansprüche 1 bis 3, wobei die Nachkommenpflanze die verbesserte Haltbarkeit und Ethylenunempfindlichkeit nach Anspruch 1 aufweist.

5. Vermehrungsmaterial einer Pflanze nach Anspruch 1, 2 oder 4, das zur geschlechtlichen Vermehrung geeignet ist, wobei eine aus dem Vermehrungsmaterial gezüchtete oder regenerierte Pflanze die verbesserte Haltbarkeit und Ethylenunempfindlichkeit nach Anspruch 1 aufweist.

6. Pflanzenteil einer Pflanze nach Anspruch 1, 2 oder 4, geeignet zur vegetativen Vermehrung, wobei eine aus dem Pflanzenteil gezüchtete oder regenerierte Pflanze die verbesserte Haltbarkeit und Ethylenunempfindlichkeit nach Anspruch 1 aufweist.

## Revendications

1. Plant mutant de concombre *(Cucumis sativus),* qui a un trait génétique monogénique et intermédiaire qui est responsable d'une durée de conservation améliorée des fruits du plant qui est directement lié à une sensibilité réduite du plant à l'éthylène,
dans lequel la durée de conservation améliorée est **caractérisée par** :
- des fruits de concombre récoltés après stockage en présence d'éthylène en une concentration de 5 ppm à 21 °C dans l'obscurité restant fermes après 12 jours, dans lequel la fermeté est définie en nécessitant une force moyenne de 5,1 kg/cm² lors de la mesure de la chair de fruit à différentes positions avec un pénétromètre ; et
- des fruits de concombre récoltés après stockage en présence d'éthylène en une concentration de 5 ppm à 21 °C dans l'obscurité restant visiblement exempts de croissance fongique après 12 jours ;
- des fruits de concombre récoltés après stockage en présence d'éthylène en une concentration de 5 ppm à 21 °C dans l'obscurité ayant une décoloration réduite par comparaison avec la lignée témoin négative après 8 jours ;
dans lequel le plant a une sensibilité réduite à l'éthylène lorsque le rapport entre la longueur moyenne d'hypocotyle du plant à un stade de plantule étiolée cultivé à 21 °C dans l'obscurité dans de l'air contenant 10 à 20 ppm d'éthylène et la longueur moyenne d'hypocotyle du plant dans un stade de plantule étiolée cultivé à 21 °C dans l'obscurité dans de l'air vaut au moins 0,15, de préférence 0,19 ;
dans lequel la sensibilité réduite à l'éthylène n'affecte pas l'expression sexuelle ; et
dans lequel le plant peut être obtenu en croisant un plant de concombre avec un plant cultivé à partir de semences de concombre, dont un échantillon représentatif a été déposé auprès du NCIMB sous le numéro d'accès NCIMB 41716, et en sélectionnant dans la descendance F1 du croisement des plants ayant la durée de conservation améliorée et une sensibilité réduite à l'éthylène.

2. Plant de concombre selon la revendication 1, dans lequel le plant peut être obtenu en sélectionnant dans la descendance F2 du croisement qui est obtenu après croisement de F1 avec lui-même ou avec un autre plant des plants ayant la durée de conservation améliorée et une sensibilité réduite à l'éthylène.

3. Semence d'un plant de concombre selon la revendication 1 ou 2, dans laquelle le fruit produit par le plant cultivé à partir de la semence présente la durée de conservation améliorée et l'insensibilité à l'éthylène tels que définis dans la revendication 1.

4. Descendance d'un plant de concombre ou d'une semence de concombre selon l'une quelconque des revendications 1 à 3, dans laquelle le plant de descendance a la durée de conservation améliorée et l'insensibilité à l'éthylène tels que définis dans la revendication 1.

5. Matériau de propagation d'un plant selon la revendication 1, 2 ou 4, approprié pour reproduction sexuée, dans lequel un plant cultivé ou régénéré à partir du matériau de propagation a la durée de conservation améliorée et l'insensibilité à l'éthylène tels que définis dans la revendication 1.

6. Partie de plant d'un plant selon la revendication 1, 2 ou 4, approprié pour reproduction végétative, dans lequel un plant cultivé ou régénéré à partir de la partie de plant a la durée de conservation améliorée et l'insensibilité à l'éthylène tels que définis dans la revendication 1.
